# EUROPEAN PATENT SPECIFICATION

(11) **EP 2 407 549 B1**
(45) Date of publication and mention of the grant of the patent: **09.10.2013**
(21) Application number: 10750325.2
(22) Date of filing: 27.01.2010
(51) Int. Cl.: C12Q 1/04, C12Q 1/34

(54) **DETECTION OF TRICHOMONAS AND CANDIDA**
NACHWEIS VON TRICHOMONAS UND CANDIDA
DÉTECTION DE TRICHOMONAS ET CANDIDA

(30) Priority: 10.03.2009 CN 200920088878 U
(43) Date of publication of application: 18.01.2012
(73) Proprietor: Autobio Diagnostics Co., Ltd., Henan 450016 (CN)
(72) Inventor: WANG, Zeyu, Zhengzhou Henan 450016 (CN); YANG, Hongyun, Zhengzhou Henan 450016 (CN); FU, Guangyu, Zhengzhou Henan 450016 (CN); WU, Xuewei, Zhengzhou Henan 450016 (CN); FENG, Chaojie, Zhengzhou Henan 450016 (CN); YANG, Yang, Zhengzhou Henan 450016 (CN); YANG, Jingjing, Zhengzhou Henan 450016 (CN); XIE, Yuanyuan, Zhengzhou Henan 450016 (CN); ZHENG, Yehuan, Zhengzhou Henan 450016 (CN); LI, Guilin, Zhengzhou Henan 450016 (CN); YANG, Fan, Zhengzhou Henan 450016 (CN)
(74) Representative: Kalhammer, Georg
(86) International application number: PCT/CN2010/070382
(87) International publication number: WO 2010/102529

(56) References cited:
- EP-A2- 0 556 685
- EP-A2- 1 536 018
- CN-A- 101 320 040
- CN-Y- 200 996 962
- US-A1- 2008 026 417
- A SANON ET AL.: 'N-acetyl-b-D-hexosaminidase from Trichomonas vaginalis: substrate specificity and activity of inhibitors.' BIOMED PHARMACOTHER vol. 59, April 2005, pages 245 - 248

## Description

### Technical Field

The invention relates to a method of distinguishing in a sample infection with *Trichomonas* from infection with *Candida,* comprising the detection of N-acetyl-β-D-hexosaminidase and pH determination in the sample.

### Prior Art

Three Trichomonas are known to be human parasites: *T. vaginalis,* found in vagina, urethra and prostate and cause trichomonas vaginitis; *T. hominis,* found in intestinal tract and cause intestinal trichomoniasis; and *T. tenax,* found in oral cavity, odontia incrustans and frog's burrow and cause oral trichomoniasis.

Following methods are frequently used for detection of Trichomonas: **Hanging Drop Method** in which the motility of Trichomonas is observed, but the results varied in different temperature; **Smear Staining Method,** in which treated or untreated samples are smeared, stained and observed by microscope, whereas particles with sizes comparable to those of Trichomonas and the range of the visual field may influence the observation; **Culture Method,** in which the Trichomonas in the sample is selectively multiplied, smeared and stained, which is time consuming; **Immunological Method**, using specific antibodies to detect antigens of Trichomonas, such as fluorescent antibody assay, ELISA, latex agglutination, while, up to date, no high-quality immunological kit is marketed for clinical detection of Trichomonas.

Most Candida found in human body, such as oral cavity and urogenital tract, are opportunistic pathogens. It is known that more than 85% opportunistic Candida pathogens are *C. albicans* and *C. tropicalis,* which may cause urogenital tract diseases in human.

Following methods are frequently used for detection of Candida: **Direct Smear Microscopy**, in which for female, swab sample is taken from vagina, cervical secretion, or milky white film on vaginal wall, while for male, scales are collected from skin lesions at glans penis, coronary sulcus, and foreskin. Samples are smeared and detected by microscopy for ovoid spores or mycelium. This method relies greatly on experience. The results varied among different visual fields, and can be interfered by particulates especially paraffin oil drops added for lubrication of samples; **Fungal Culture Method**, a time consuming method in which particular media such as Sabouraud agar (such as Oxoid Ltd., Basingstoke, UK) are used to selectively multiply Candida in the sample; **Latex Agglutination Method**, in which polyclonal antibodies are reacted with mannan of Candida, although no high-quality product for clinical use has be found.

Diseases caused by Trichomonas and Candida are hard to be distinguished in terms of symptom. Since therapeutic agents for them are also different, a diagnostic means that is simple, rapid and effective is needed.

CN 101320040 A describes a kit that uses distinct reagents for detecting *Trichomonas* and *Candida,* and teaches the use of N-acetyl-β-D-aminohexoside as a detecting agent for *Candida,* and a Trichomonas-specific protease substrate as a detecting agent for *Trichomonas.*

EP 1536018 A2 discloses a culture medium for the specific identification and/or differentiation of Candida albicans and Candida tropicalis yeast.

Sanon et al. (2005) Biomedicine & Pharmacotherapy 59, 245-248 investigates some biochemical characteristics of N-acetyl-β-D-hexosaminidase purified from *Trichomonas vaginalis,* including substrate specificity and activity of inhibitors.

### Summary of Invention

The present invention is defined in the claims.

An aspect of the disclosure relates to a kit, especially:
1.1 A kit comprising N-acetyl-aminohexoside as a Trichomonas detecting agent.
1.2 The kit according to 1.1, further comprising an pH indicator.
1.3 The kit according to 1.2, wherein N-acetyl-β-D-aminohexoside is further optionally a Candida detecting agent.
1.4 The kit according to any preceding items, wherein said N-acetyl-β-D-aminohexoside is N-acetyl-β-D-glucosaminide or N-acetyl-β-D-aminogalactoside.
1.5 The kit according to any preceding items, wherein said N-acetyl-β-D-aminohexoside carries a chromogen, preferably the chromogen being bromoindolyl, p-nitrophenyl, 4-methylumbelliferone or naphthol group.
1.6 The kit according to any preceding items, further comprising at least one agent selected from the group consisting of neuraminidase detecting agent, prolidase detecting agent, hydrogen peroxide detecting peroxidase agent, and leukocyte esterase detecting agent.
1.7 The kit according to any preceding items, further comprising a standard color chart, such as NAG standard color chart and/or pH standard color chart.
1.8 The kit according to any preceding items, wherein at least one agent selected from N-acetyl-β-D-aminohexoside, neuraminidase detecting agent, prolidase detecting agent, peroxidase agent, and leukocyte esterase detecting agent, and pH indicator is immobilized on a solid phase carrier, preferably the solid phase carrier being selected from plastics and paper of adsorption capacity such as rag paper, wood pulp paper, bagasse paper and fiber glass filter.
1.9 The kit according to any preceding items which is a combined detection panel comprising a panel basal means and a sealing means disposed on the panel basal means.
1.10 The kit according to 1.9, wherein said sealing means is a sealing cap buckled on the slot side of the reaction zone on the panel basal means.
1.11 The kit according to 1.9, wherein said sealing means is a sealing strip attached to the slot side of the reaction zone on the panel basal means.

Another aspect of the disclosure relates to a use, especially:
2.1 Use of N-acetyl-β-D-aminohexoside for the detection of Trichomonas.
2.2 Use of N-acetyl-β-D-aminohexoside in combination with an pH indicator for determination of presence of Trichomonas or Candida.
2.3 Use of N-acetyl-β-D-aminohexoside for the preparation of a kit for detecting Trichomonas.
2.4 Use of N-acetyl-β-D-aminohexoside in combination with an pH indicator for the preparation of a kit which is used for determination of presence of Trichomonas or Candida.
2.5 The use according to any preceding items, wherein said N-acetyl-β-D-aminohexoside is N-acetyl-β-D-aminoglucoside or N-acetyl-β-D-aminogalactoside.
2.6 The use according to any preceding items, wherein said N-acetyl-β-D-aminohexoside carries a chromogen, preferably the chromogen being bromoindolyl, p-nitrophenyl, 4-methylumbelliferone or naphthol group.
2.7 The use according to any preceding items, wherein said N-acetyl-β-D-aminohexoside is immobilized on a solid phase carrier, preferably the solid phase carrier being selected from plastics and paper of adsorption capacity such as rag paper, wood pulp paper, bagasse paper and fiber glass filter.

A further aspect of the disclosure relates to a detection method, especially:
3.1 A method for the detection of presence of Trichomonas in a sample, comprising contacting N-acetyl-β-D-aminohexoside with the sample.
3.2 A method for the detection of presence of Trichomonas in a sample, comprising contacting N-acetyl-β-D-aminohexoside with the sample, and contacting an pH indicator with the sample.
3.3 The method according to any preceding items, wherein said sample is a sample from vagina, urethra, prostate, or oral cavity.
3.4 The method according to any preceding items, wherein said N-acetyl-β-D-aminohexoside is N-acetyl-β-D-aminoglucoside or N-acetyl-β-D-aminogalactoside.
3.5 The method according to any preceding items, wherein said carries a chromogen, preferably the chromogen being bromoindolyl, p-nitrophenyl, 4-methylumbelliferone or naphthol group.
3.6 The method according to any preceding items, wherein said N-acetyl-β-D-aminohexoside is immobilized on a solid phase carrier, preferably the solid phase carrier being selected from plastics and paper of adsorption capacity such as rag paper, wood pulp paper, bagasse paper and fiber glass filter.
3.7. The method according to any preceding items, wherein negative reaction to N-acetyl-β-D-aminohexoside is determined as free of Trichomonas or Candida, or positive reaction to N-acetyl-β-D-aminohexoside with pH of no less than 4.8 is determined as presence of Trichomonas, or positive reaction to N-acetyl-β-D-aminohexoside with pH of no more than 4.6 is determined as presence of Candida.
3.8 A combined detection method, comprising a method according to any preceding items, together with at least one detection selected from the group consisting of neuraminidase detection, prolidase detection, hydrogen peroxide detection, and leukocyte esterase detection.
3.9 The combined detection method according to 3.8, wherein at least one of said neuraminidase detection, prolidase detection, hydrogen peroxide detection, and leukocyte esterase detection is conducted on a solid phase carrier.
3.10 The method according to any preceding items, wherein the result of at least one reaction selected from N-acetyl-β-D-hexosaminidase reaction, neuraminidase reaction, prolidase reaction, hydrogen peroxide reaction, leukocyte esterase reaction and pH indication is determined by comparing the color of the sample with corresponding standard color chart, or by reading the color of the sample by a reader.

### Detailed Description of Invention

### Enzyme

As described herein, N-acetyl-β-D-hexosaminidase means an enzyme classified as EC. 3.2.1.52, according to the designation of Nomenclature Committee of the International Union of Biochemistry. It mainly acts on N-acetyl-β-D-aminoglucoside substrate and N-acetyl-β-D-aminogalactoside substrate, hydrolyzing the terminal non-reducing N-acetyl-D-hexosamine of N-acetyl-β-D-aminohexoside. This enzyme is an inducible enzyme: addition of analog of the substrates of said enzyme can induce secretion of this enzyme. As described herein, an enzyme inducer refers to a substance that can induce the secretion of N-acetyl-β-D-hexosaminidase according to the invention and may be selected from galactose, glucose, fructose, and the like.

This enzyme is also known as hexosaminidase, β-acetylaminodeoxyhexosidase, *N*-acetyl-β-D-hexosaminidase, *N*-acetyl-beta-hexosaminidase, β-hexosaminidase, β-acetylhexosaminidinase, β-D-*N-*acetylhexosaminidase, β-*N*-acetylglucosaminidase, β-*N*-acetyl-D-hexosaminidase, hexosaminidase A, *N*-acetylhexosaminidase, and β-D-hexosaminidase, all of which are contemplated in the invention.

It is known that both *C. albica* and *C. tropica* have this enzyme. Chinese Patent 200720089307.9 describes use of N-acetylhexosaminase method for the detection of colpitis mycotica (also referred to as candidal vaginitis), the content of which is incorporated herein by reference in its entirety. Up to now, no clinical sample before purification is reported to be Trichomonas-positive after detection with this enzyme.

The inventor has surprisingly discovered that Trichomonas and most members of Candida can be detected by detecting N-acetyl-β-D-hexosaminidase.

### Substrate

The compound that is the substrate of the N-acetyl-β-D-hexosaminidase described herein is known as β-acetylaminohexoside, *N*-acetyl-β-D-aminohexoside, *N*-acetyl-beta-aminohexoside, β-aminohexoside, β-D-*N-*acetylaminohexoside, β-*N*-acetylaminoglucoside, β-*N*-acetyl-D-aminohexoside, N-acetylaminohexoside, or β-D-aminohexoside. When the compounds designated as such are used as substrates of the N-acetyl-β-D-hexosaminidase according to the invention, they are all included in the scope of the invention. In some embodiments, N-acetyl-β-D-hexosaminidase is shortened as acetylaminoglucoside or abbreviated as NAG.

In some embodiments, the N-acetyl-β-D-aminohexoside according to the invention is N-acetyl-β-D-aminoglucoside or N-acetyl-β-D-aminogalactoside.

The N-acetyl-β-D-aminohexoside substrate according to the invention carries chromogen. The chromogen is preferably bromoindolyl (such as 5-bromo-4-chloro-3-indolyl), p-nitrophenyl, 4-methylumbelliferone or naphthol group. Such substrates include, but not are not limited to, 5-bromo-4-chloro-3-indolyl-acetylaminoglucoside, p-nitrophenyl-acetylaminoglucoside (PNP-NAG), p-nitrophenyl-acetylaminogalactoside, and the like. Such compound can be purchased from many companies. When the chromogen is p-nitrophenyl or naphthol group, a stopping solution is added into the reaction system in order to terminate the color development in time.

### pH Value

It is known in the art that Trichomonas are anaerobic organisms that produces a large amount of lower organic amines in their metabolism. The inventor has found that pH of clinical samples containing Trichomonas usually increases significantly and reaches pH 5.0 or even higher.

It is also known in the art that pH of sample containing Candida is usually lower than pH 4.5. The inventor has made statistical analysis on 1362 clinical samples and found that 94.8% of samples infected with Candida has a pH equal to or lower than 4.6.

The inventor noticed co-existent Candida- and Trichomonas-infections which are represented as acetylhexosaminidase-positive reaction and a pH equal to or slight lower than 4.6. However, the occurrence of this situation is extremely low, even lower than that of a Candida-infected sample with pH higher than 4.6, as discovered by the inventor. Therefore, the inventor believes that combination of N-acetyl-β-D-hexosaminidase detection with pH determination can be used to distinguish samples infected with Trichomonas from samples infected with Candida.

### Detection Method

Accordingly, the disclosure relates to a method for the detection of presence of Trichomodas in a sample, comprising contacting N-acetyl-β-D-aminohexoside with the sample. N-acetyl-β-D-aminohexoside, as a substate of N-acetyl-β-D-hexosaminidase, can detect presence of said enzyme in the sample. More specifically, the method for the detection of Trichomonas comprises contacting chromogen-containing N-acetyl-β-D-aminohexoside detecting agent with the sample and allowing color development. The method for the detection of Trichomonas also comprises determining pH of the sample.

The invention also relates to a method for the selective detection of presence of Trichomonas and Candida in a sample, comprising contacting N-acetyl-β-D-aminohexoside with the sample and contacting an pH indicator with the sample so as to determine whether there exists Trichomonas and/or Candida in the sample. N-acetyl-β-D-aminohexoside can detect presence of N-acetyl-β-D-hexosaminidase in the sample. The pH indicator can detect the acidity or alkalinity of the sample.

In the method according to the invention, there is no particular limit to the sequence of the enzyme detection step and the pH detection step, and they can also be performed simultaneously.

In the method according to the invention, the sample comprises those taken from vagina, urethra, prostate or oral cavity. For example, vaginal sample can be a leukorrheal sample, such as a sample taken from posterior fornix. The sample can be diluted with diluent such as physiological saline. The sample to be contacted with the pH indicator can be used without dilution.

In a specific embodiment, a sample is tested in the method according to the invention, and is determined to be free of Trichomonas- and Candida-infection if it is N-acetyl-β-D-hexosaminidase-negative, or is determined to be infected with Trichomonas or Candida if it is N-acetyl-β-D-hexosaminidase-positive. In the later situation, the sample is further determined to be infected with Trichomonas if its pH is no less than 5.0 (preferably no less than 4.8), or the sample is further determined to be infected with Candida if its pH is no more than 4.5 (preferably no more than 4.6).

### Kit

The disclosure also relates to a kit comprising N-acetyl-β-D-aminohexoside as an agent for detecting Trichomonas. The disclosure also relates to a kit comprising N-acetyl-β-D-aminohexoside as an agent for detecting both Trichomonas and Candida. When N-acetyl-β-D-hexosaminidase activity is detected, Trichomonas and/or Candida is determined to be present in the sample.

The kit may also comprise an pH indicator, such as at least one of the many pH indicators known in the art, especially an pH indicator that can distinguish pH 4.6 and pH 4.8. For example, such an pH indicator may be bromocresol green, chlorophenol red, propyl red, and the like.

In some embodiments, the kit may comprise a standard color chart, including a standard color chart for enzymatic reaction (such as a standard color chart for NAG enzyme reactions, and other enzyme reactions mentioned hereinafter) and/or a standard color chart for pH test.

In further embodiments, the kit may be equipped with a sensor that can sense color change, such as a sensor that can sense the color change of a NAG-detecting agent, and/or a sensor that can sense change of pH indicator, or the kit may be provided with a device connected to electronic devices such as a computer. The change caused by contact of NAG-detecting agent and/or pH indicator with the sample may be captured by said sensor. A signal may be transmitted from the sensor to said electronic device. A program run in the electronic device may recognize the signal and give a result of the NAG reaction and/or pH test. Therefore, the kit may also comprise computer readable medium, such as a CD, for installation of corresponding recognition and analysis software.

Moreover, the kit may comprise a reagent for sample dilution, such as physiological saline, and/or a reagent for reaction termination, such as 5-10 mg/ml NaOH aqueous solution.

### Solid Phase

In some embodiments, the detection according to the invention can be completely or partially performed on a solid phase. For this purpose, the detecting agent and/or substance according to the invention may be immobilized on a solid phase carrier. For example, N-acetyl-β-D-aminohexoside can be immobilized on one part of the solid phase carrier, and the pH indicator can be immobilized on another part of the solid phase carrier; alternatively, only N-acetyl-β-D-aminohexoside is immobilized on the solid phase carrier and the pH indicator is not immobilized.

Said solid phase carrier may be paper of adsorption capacity, for example, filter paper, such as rag paper, wood pulp paper, bagasse paper and fiber glass filter; it may also be polyester membrane, nylon membrane *etc*.; and it may also be plastics of adsorption capacity.

Accordingly, the kit can be a solid phase detection means, such as a detection panel, a detection strip, a detection block, and the like. The solid phase carrier can further be horizontally covered by or internally embedded in a plastics basal means.

### Preparation of Kit

The disclosure also relates to a method for the preparation of the kit described herein, wherein the preparation of N-acetyl-β-D-aminohexoside detection zone comprises adding solution of N-acetyl-β-D-aminohexoside containing chromogen to a solid phase carrier of adsorption capacity, or immersing the solid phase of adsorption capacity in said solution, then subjecting it to drying such as vacuum drying, heated drying, dehumifying drying, and the like. Preferably, said solution of chromogen-containing NAG is a 1-3 mg/ml solution of chromogen-containing NAG in phosphate buffered saline (PBS). In a specific embodiment, said chromogen-containing NAG in PBS solution is prepared as follows: the chromogen-containing NAG is dissolved in organic solvents such as methanol, ethanol, dimethyl sulfoxide (DMSO) or dimethylformamide (DMF), then dissolved in a 10-50 mM PBS buffer (pH 5.0-6.0) containing the enzyme inducer described above, and mixed thoroughly.

In a specific embodiment of the kit, bromocresol green is used as a pH indicator. Bromocresol green can be dissolved in absolute ethanol and mixed thoroughly to yield a 1-3 mg/ml solution. Furthermore, the prepared bromocresol green solution may be added to the solid phase carrier such as a piece of paper, or a piece of paper may be immersed in said bromocresol green solution and dried (such as by vacuum drying, heated drying, dehumifying drying, and the like.) to yield the pH value detection paper.

### Use

The disclosure also relates to use of N-acetyl-β-D-aminohexoside for the detection of Trichomonas. The disclosure also relates to use of N-acetyl-β-D-aminohexoside for the preparation of a kit for detecting Trichomonas. More specifically, the disclosure relates to use of N-acetyl-β-D-aminohexoside for the preparation of a means for detecting Trichomonas.

The disclosure also relates to use of N-acetyl-β-D-aminohexoside in combination with an pH indicator for detection of Trichomonas or for selective detection of Trichomonas and Candida. The disclosure also relates to use of N-acetyl-β-D-aminohexoside in combination with an pH indicator for the preparation of a kit for determination of presence of Trichomonas and/or Candida.

### Combination with Other Methods and Kits for Detection of Vaginitis

Factors other than Trichomonas and Candida may cause diseases in female vagina. Common examples are bacterial vaginosis (briefly BV), also called nonspecific vaginitis, Gardnerella vaginitis, Haemophilus vaginalis vaginitis, which can be diagnosed by detecting generation of H₂O₂ and activity of neuraminidase and prolidase. Besides bacterial vaginosis, candidal vaginitis (also referred to as colpitis mycotica), trichomonas vaginitis, other vaginitis with unknown cause may be diagnosed by detecting leukocyte esterase activity. See, for example, Chinese Patent 200720080307.9.

Therefore, the method and kit for detecting Trichomonas and/or Candida can further be combined with the current other method and kit for detecting other vaginitis (such as bacterial vaginosis and vaginitis with unknown cause) in the art to generate simple and convenient products such as combined detection panels, which will facilitate not only medical institutions but also those final users to rapidly, thoroughly and efficiently determine the property of the vaginal disease and promptly select effective medicaments for treatment of the disease.

For this purpose, the kit can additionally comprise other detecting agents, such as agents known for vaginal disease detection, such as neuraminidase detecting agent, prolidase detecting agent, hydrogen peroxide detecting agent, and leukocyte esterase detecting agent, and the like.

In a specific embodiment, at least one of said neuraminidase detecting agent, prolidase detecting agent, hydrogen peroxide detecting agent, and leukocyte esterase detecting agent is immobilized on a solid phase carrier. For example, all the agents may be immobilized on a solid phase carrier. One example of immobilization is adding a solution of the detecting agents to the solid phase carrier, or immersing the solid phase carrier in said solution, followed by drying by means of vacuum, heating, dehumidification, and the like.

For the aforementioned purposes, the disclosure relates to a combined detection panel comprising a panel basal means and a sealing means disposed on the panel basal means, said panel basal means being separately equipped with N-acetylhexosaminidase reaction zone and a reaction zone with pH reagent filled therein, wherein N-acetylhexosaminidase substrate is immobilized in the N-acetylhexosaminidase reaction zone.

Said panel basal means may optionally set a neuraminidase reaction zone and a prolidase reaction zone, wherein neuraminidase substrate is immobilized in the neuraminidase reaction zone and prolidase substrate is immobilized in the prolidase reaction zone.

Said panel basal means may optionally set a hydrogen peroxide reaction zone, wherein peroxidase is immobilized.

Said panel basal means may optionally set a leukocyte esterase reaction zone, wherein leukocyte esterase substrate is immobilized.

Said sealing means is a sealing cap buckled on the slot side of the reaction zone on the panel basal means, or a sealing strip attached to the slot side of the reaction zone on the panel basal means. Other forms that are suitable for liquid (such as drops) loading may be employed.

An aspect of the disclosure relates to a combined detection method comprising the Trichomonas detection and/or Candida detection as described above, and further comprising at least one detection selected from the group consisting of neuraminidase detection, prolidase detection, hydrogen peroxide detection and leukocyte esterase detection, and the like. Said neuraminidase detection, prolidase detection, hydrogen peroxide detection and leukocyte esterase detection are all known in the art. Accordingly, in a specific embodiment, at least one of said neuraminidase detection, prolidase detection, hydrogen peroxide detection and leukocyte esterase detection is performed on a solid phase carrier, for example, all are performed on a solid phase carrier.

In a specific embodiment, five vaginal biochemical marker: N-acetylhexosaminidase, neuraminidase, prolidase, hydrogen peroxide and leukocyte esterase are integrated in a combined detection panel with pH detection. These markers are tested together to facilitate the diagnosis of the health condition of the female vagina.

Vaginal secretion is taken according to conventional means, to which a sample processing solution may be added for processing. The sample is dropped into each reaction zone of the combined detection panel, mixed thoroughly and allowed for color development, and, if desired, supplemented with color developing solution for color development. The presence or type of vaginosis can be determined by combining the pH value of the sample with the change of the color and/or clarity of reaction in the slots. For example, the N-acetylhexosaminidase reaction appears yellow under alkaline conditions; the prolidase reaction appears yellow; the neuraminidase reaction appears bright red or purple under action of color developing solution; hydrogen peroxide under catalysis of peroxidase can oxide chromogenic substrate to generate a red product; and the leukocyte esterase appears blue. The darkness of the color appeared is proportional to the activity of each enzyme or hydrogen peroxide concentration. In practical testing, some reaction zone is supplemented with corresponding color developing agent or pH adjusting agent.

As described above, a combined detection panel including merely a N-acetylhexosaminidase reaction zone and an pH reagent reaction zone, with corresponding reagents immobilized in slots, is enough to distinguish Trichomonas- and Candida-infection. Further addition of reaction zones for neuraminidase, prolidase, hydrogen peroxide and/or leukocyte esterase on the combined detection panel facilitate detecting these biochemical markers of vagina together so as to further distinguish bacterial vaginosis, colpitis mycotica, trichomonas vaginitis and vaginitis of other causes.

### Example

### Example 1 Preparation of detection paper

p-nitrophenyl N-acetyl-β-D-aminoglucoside (purchased from Shanghai Kayon Biological Technology Co. Ltd., Catolog No. M0110) was dissolved in methanol, and then dissolved in 25 mM D-galactose-containing phosphate buffered saline (pH 5.5) to a concentration of 1 mg/ml and mixed thoroughly. The NAG solution was added to 5mm-wide pieces of paper at 15 ul/piece and dried overnight to yield the NAG detection paper.

Sodium hydroxide was dissolved in pure water to reach a concentration of 8 mg/ml and mixed thoroughly to yield the stopping solution.

Bromocresol green was dissolved in absolute ethanol to reach a concentration of 2 mg/ml and mixted thooughly. It was added to 5 mm-wide pieces of paper at 15 ul/piece and dried overnight to yield the pH detection paper.

### Example 2 pH value detection

Up to 2009, more than 10000 samples of leukorrheal samples had been obtained from outpatients visiting gynecology department of the third hospital affiliated with Zhengzhou University. pH value of these samples were detected with pH detection paper prepared above. The results showed that samples with Trichomonas infection all have a pH of no less than 4.8, while samples with Candida infection all have a pH of no more than 4.6.

### Example 3 Enzyme detection in combination with pH detection

Samples were taken by sterile swab from posterior fornix of each of 212 outpatients visiting gynecology department of the third hospital affiliated with Zhengzhou University.

### 1. The detection according to the invention

Samples were directly dipped to the pH detection paper prepared above for pH reading, and then diluted with physiological saline and dropped to the NAG detection paper prepared above. The NAG detection paper was heated at 37°C for 15 minutes, and a stopping reagent solution was added dropwise. The NAG detection paper appeared yellow, suggesting presence of N-acetyl-β-D-hexosaminidase activity.

### 2. Conventional detection

Two other samples were taken and cultured on Candida chromogenic media (HiCrome Candida Differential Agar, Himedia Laboratories Pvt. Limited, India) and Trichomonas media (Trichomonas Medium, OXOID Limited, United Kingdom) following manufacturer's instruction.

### Result

1. Trichomonas:
   (1) Sensitivity: Among 9 samples that were determined to be Trichomonas-positive by the culturing method, 8 samples were also determined to be positive by the combined detection method according to the invention. The sensitivity of the method according to the invention for Trichomonas detection is 88.9% (8/9).
   (2) Specificity: Among 203 samples that were determined to be Trichomonas-negative by the culturing method, 198 samples were also determined to be negative by the combined detection method according to the invention. The specificity of the method according to the invention for Trichomonas detection is 97.7% (198/203).
2. Candida:
   (1) Sensitivity: Among 34 samples that were determined to be Candida-positive by the culturing method, 26 samples were also determined to be positive by the combined detection method according to the invention. The sensitivity of the method according to the invention for Trichomonas detection is 76.5% (26/34).
   (2) Specificity: Among 178 samples that were determined to be Candida-negative by the culturing method, 149 samples were also determined to be negative by the combined detection method according to the invention. The specificity of the method according to the invention for Trichomonas detection is 85.6% (149/178).

### Example 4 Preparation of a combined detection kit

NAG detection paper and pH detection paper were prepared as described in Example 3. Conventional reagents for detecting neuraminidase, prolidase, hydrogen peroxide and leukocyte esterase were individually adsorbed to different pieces of paper according to known method in the art. These pieces were separately fixed on a plastic panel or attached to a long paper strip.

### Example 5 Gynecological combined detection

### Protocol 1:

Vaginal secretion was sampled, diluted, and dropped onto the various pieces of paper prepared in Example 4, with color developing solution dropped onto the neuraminidase paper.

After 1 minute, pH was read by comparing with the standard color chart.

Then the whole plastic panel or the long paper strip was incubated at 37°C for 15 minutes.

The stopping solution was dropped onto the NAG paper. After 30 seconds, results other than pH were read.

### Protocol 2:

It differed from Protocol 1 merely in that pH is read using a reader. For this reason, the sample was not loaded onto the pH detection zone before the incubation. After incubation, the diluted or undiluted sample was loaded.

### Results:

For the hydrogen peroxide paper, no or light color development indicates positive, otherwise negative. For NAG paper, neuraminidase paper, prolidase paper and leukocyte esterase paper, no or light color development indicates negative, otherwise positive.

### Discussion:

For example, if the N-acetylhexosaminidase paper appears positive, trichomonas vaginitis is diagnosed if the paper immobilized with pH reagent shows pH≥4.8, while colpitis mycotica is diagnosed if pH≤4.6. If the N-acetylhexosaminidase paper appears negative and the prolidase paper appears positive, it may indicate BV. If the neuraminidase paper appears positive, it may be BV. If the hydrogen peroxide paper appears positive, it indicates vaginal flora imbalance and may be BV. If all the paper mentioned above appears negative, except that the leukocyte esterase paper appears positive, it indicates other types of vaginitis.

## Claims

1. A method of distinguishing in a sample infection with *Trichomonas* from infection with *Candida,* comprising the detection of N-acetyl-β-D-hexosaminidase and pH determination in the sample.

2. The method of claim 1, wherein said sample is determined to be infected with *Trichomonas* if (i) it is N-acetyl-β-D-hexosaminidase-positive and (ii) its pH is no less than 4.8.

3. The method of claim 1, wherein said sample is determined to be infected with *Candida* if (i) it is N-acetyl-β-D-hexosaminidase-positive and (ii) its pH is no more than 4.6.

## Patentansprüche

1. Verfahren zur Unterscheidung einer Infektion mit *Trichomonas* von einer Infektion mit *Candida* in einer Probe, umfassend die Detektion von N-Acetyl-β-D-hexosaminidase und die pH-Bestimmung in der Probe.

2. Verfahren nach Anspruch 1, wobei die Probe als mit *Trichomonas* infiziert bestimmt wird, wenn sie (i) N-Acetyl-β-D-hexosaminidase-positiv ist und (ii) ihr pH nicht weniger als 4,8 beträgt.

3. Verfahren nach Anspruch 1, wobei die Probe als mit *Candida* infiziert bestimmt wird, wenn sie (i) N-Acetyl-β-D-hexosaminidase-positiv ist und (ii) ihr pH nicht mehr als 4,6 beträgt.

## Revendications

1. Procédé pour opérer une distinction dans un échantillon entre une infection avec *Trichomonas* et une infection avec *Candida*, comprenant la détection de la N-acétyl-β-D-hexosaminidase et la détermination du pH dans l'échantillon.

2. Procédé selon la revendication 1, dans lequel on détermine une infection dudit échantillon avec *Trichomonas* lorsque (i) celui-ci est positif vis-à-vis de la N-acétyl-β-D-hexosaminidase et (ii) son pH n'est pas inférieur à 4,8.

3. Procédé selon la revendication 1, dans lequel on détermine une infection dudit échantillon avec *Candida* lorsque (i) celui-ci est positif vis-à-vis de la N-acétyl-β-D-hexosaminidase et (ii) son pH n'est pas supérieur à 4,6.
